# EUROPÄISCHE PATENTSCHRIFT

(11) **EP 2 893 946 B1**
(45) Veröffentlichungstag und Bekanntmachung des Hinweises auf die Patenterteilung: **05.04.2017**
(21) Anmeldenummer: 15150522.9
(22) Anmeldetag: 23.02.2012
(51) Int. Cl.: A61M 5/28, A61M 5/31

(54) **Verschluss für eine Pulverspritze und Pulverspritze**
Closure piece for a powder syringe, and powder syringe
Bouchon pour seringue de poudre et seringue de poudre

(30) Priorität: 03.03.2011 DE 102011013792
(43) Veröffentlichungstag der Anmeldung: 15.07.2015
(62) Teilanmeldung aus: 12707475.5
(73) Patentinhaber: Vetter Pharma-Fertigung GmbH & Co. Kg, 88212 Ravensburg (DE)
(72) Erfinder: Glocker, Joachim, 88250 Weingarten (DE); Roedle, Tilman, 88364 Wolfegg (DE)
(74) Vertreter: Gleiss, Alf-Olav

(56) Entgegenhaltungen:
- EP-A1- 0 072 057
- WO-A2-03/070296
- JP-A- H0 910 281
- US-A- 4 331 146

## Beschreibung

Die Erfindung betrifft einen Verschluss für eine Pulverspritze gemäß Oberbegriff des Anspruchs 1 sowie eine Pulverspritze gemäß Oberbegriff des Anspruchs 16.

Verschlüsse und Pulverspritzen der hier angesprochenen Art sind bekannt. Bevorzugt kommen als Pulverspritzen Doppelkammersysteme zur Anwendung, welche eine proximale Kammer und eine distale Kammer aufweisen. Die proximale Kammer umfasst typischerweise ein flüssiges Lösungsmittel und ist durch einen Mittelstopfen von der distalen Kammer getrennt, welche ein in dem Lösungsmittel lösliches Material aufweist. Dies kann ein Pulver sein, welches eine Rieselneigung aufweist. Bekannte Verschlüsse weisen einen Kanal auf, der einerseits mit der distalen Kammer in Fluidverbindung steht und andererseits mit einer auf den Verschluss aufgesetzten Kanüle oder Spritzennadel in Fluidverbindung gebrachte werden kann. Es ist möglich, dass Pulver in den Bereich des in den Verschluss vorgesehenen Kanals beziehungsweise in den Kanal selbst gelangt. Dabei können dort Agglomerate des pulverförmigen Materials entstehen, welche gegebenenfalls nicht mehr lösbar sind. Der Kanal ist dann verstopft, so dass das Doppelkammersystem nicht mehr verwendet werden kann. Das gleiche Problem besteht auch bei einer Pulverspritze, die nicht als Doppelkammersystem ausgebildet ist, aber einen Verschluss mit einem Kanal aufweist. Auch in diesem Fall können sich in dem Kanal Pulveragglomerate bilden, welche diesen verstopfen.

Ein aus dem Stand der Technik bekannter Verschluss für eine Pulverspritze ist in WO03/070296 A2 offenbart.

Aufgabe der Erfindung ist es daher, einen Verschluss für eine Pulverspritze sowie eine Pulverspritze zu schaffen, welche die beschriebenen Nachteile nicht aufweist.

Die Aufgabe wird gelöst, indem ein Verschluss mit den Merkmalen des Anspruchs 1 geschaffen wird. Der Verschluss für eine Pulverspritze umfasst einen Grundkörper und ein Dichtelement, welches an dem Grundkörper so angeordnet ist, dass es an einer distalen Öffnung einer Pulverspitze dichtend anliegt, wenn der Verschluss auf der Pulverspritze in seiner Verschließposition angeordnet ist. Der Verschluss weist außerdem einen Kanal auf, welcher den Grundkörper und das Dichtelement durchsetzt. Dieser weist ein proximales und ein distales Ende auf. Der Verschluss zeichnet sich durch ein Rückhalteelement aus, welches so ausgebildet und/oder anordenbar ist, dass vor einer Aktivierung der Pulverspritze ein Eindringen von Pulver aus einer Kammer der Pulverspritze in den Kanal zumindest weitgehend, vorzugsweise vollständig verhindert wird, wenn der Verschluss in seiner Verschließposition auf der Pulverspritze angeordnet ist. Eine zumindest weitgehende Verhinderung spricht an, dass höchstens so viel Pulver in den Kanal eindringen kann, dass eine Agglomeratbildung ausgeschlossen ist. Das Rückhalteelement ist entweder so ausgebildet, dass es ein Eindringen des pulverförmigen Materials in den Kanal des Verschlusses verhindert, wenn dieser in seiner Verschließposition angeordnet ist, oder es kann - gegebenenfalls auch getrennt von den restlichen Elementen des Verschlusses - so angeordnet werden, dass es ein Eindringen des Pulvers in den Kanal verhindert. Bevorzugt wird das Rückhalteelement so angeordnet, dass Restvolumina, in denen eine Agglomeratbildung möglich ist, minimiert oder besonders bevorzugt vollständig vermieden werden. Das Rückhalteelement kann auch zugleich entsprechend ausgebildet und entsprechend anordenbar sein. Durch das Rückhalteelement wird der Kanal quasi von der Kammer, in welcher das Pulver vorliegt, getrennt, so dass sich in dem Kanal keine Agglomerate bilden können, welche denselben verstopfen könnten. Die Funktion einer mit dem Verschluss ausgestatteten Pulverspritze ist daher auch über eine lange Lagerzeit gewährleistet. Es muss auch nicht darauf geachtet werden, in welcher Lage eine mit dem Verschluss verschlossene Pulverspritze gelagert wird, weil aufgrund des Rückhalteelements in keiner Lage Pulver oder zumindest keine für eine Agglomeratbildung ausreichenden Pulvermenge in den Kanal eindringen kann.

Bevorzugt wird ein Verschluss, der eine Verschlusskappe zum Verschließen des distalen Endes des Kanals umfasst, wobei die Verschlusskappe einen stabförmigen Vorsprung aufweist. Dieser durchsetzt den Kanal, wobei an ihm das Rückhalteelement vorgesehen ist. Der stabförmige Vorsprung verschließt quasi den Kanal. Dies hat den Vorteil, dass bei einer Entfernung der Verschlusskappe zur Aktivierung der Pulverspritze zugleich auch der Kanal freigegeben wird, ohne dass es weiterer Maßnahmen bedarf. Außerdem sind bei einem Verschluss, welcher ohnehin eine Verschlusskappe umfasst, keine weiteren Elemente nötig.

Bevorzugt wird auch ein Verschluss, bei dem das Rückhalteelement als vorzugsweise geschlitzte Membran ausgebildet ist. Die Membran kann durch die beim Aktivieren der Spritze beziehungsweise zur Abgabe des Spritzeninhalts an einen Patienten aufgebauten Druckkräfte aufgesprengt, verformt und mit Hilfe einer nadelförmigen Vorrichtung aufgestochen oder im Bereich eines Schlitzes aufgeweitet werden, um den Kanal freizugeben.

Es wird auch ein Verschluss bevorzugt, bei dem das Rückhalteelement als entlang des Kanals verlagerbares Element ausgebildet ist. Bevorzugt ist dieses quasi im Lagerzustand der Spritze an dem proximalen Ende des Kanals angeordnet und verschließt diesen. Zur Aktivierung der Pulverspritze kann das Element entlang des Kanals zu dessen distalem Ende verlagert werden, wo es schließlich aus dem Kanal austritt und damit denselben freigibt.

Bevorzugt wird auch ein Verschluss, bei dem das Rückhalteelement eine lösliche Substanz umfasst. Besonders bevorzugt umfasst das Rückhaltement eine lyophilisierte Substanz. Diese kann beim Aktivieren der Spritze durch ein Lösungsmittel gelöst werden, so dass der Kanal freigegeben wird.

Schließlich wird noch ein Verschluss bevorzugt, bei welchem das Rückhalteelement eine Verschlussscheibe umfasst, die so ausgebildet ist, dass sie an einer Verengung einer Pulverspritze dichtend anliegen kann. Auf diese Weise kann sie ein Eindringen von Pulver in den Kanal verhindern. Die Verschlussscheibe ist insbesondere im Bereich der Verengung der Pulverspritze so anordenbar, dass vor einer Aktivierung der Pulverspritze kein Pulver in den Kanal eindringen kann. Bei einer Aktivierung der Pulverspritze wird die Verschlussscheibe von der Verengung wegverlagert, so dass ein Fluidpfad zu dem Kanal freigegeben wird.

Weitere Ausgestaltungen ergeben sich aus den Unteransprüchen.

Die Aufgabe wird ebenfalls gelöst, indem eine Pulverspritze mit den Merkmalen des Anspruchs 16 geschaffen wird. Diese weist eine distale Öffnung auf und zeichnet sich dadurch aus, dass sie einen Verschluss nach einem der Ansprüche 1 bis 15 trägt. Aufgrund des Verschlusses, der ein Rückhalteelement für das Pulver umfasst, kann die Pulverspritze lange und in beliebiger Lage gelagert werden, ohne dass die Gefahr einer Agglomeratbildung von Pulver in dem Kanal besteht, weil das Pulver durch das Rückhalteelement zurückgehalten wird.

Bevorzugt ist die Pulverspritze als Doppelkammersystem ausgebildet.

Besonders bevorzugt wird eine Pulverspritze, die eine Verengung im Bereich ihres distalen Endes aufweist.

Die Erfindung wird im Folgenden anhand der Zeichnung näher erläutert. Es zeigen:
- Figur 1: eine als Doppelkammersystem ausgebildete Pulverspritze mit einem ersten Ausführungsbeispiel des Verschlusses;
- Figur 2: eine Detailansicht des Ausführungsbeispiels eines Verschlusses gemäß Figur 1;
- Figur 3: ein zweites Ausführungsbeispiel eines Verschlusses;
- Figur 4: eine Schnittansicht des Ausführungsbeispiels gemäß Figur 3 entlang der Linie A-A;
- Figur 5: ein drittes Ausführungsbeispiel eines Verschlusses;
- Figur 6: ein in Hinblick auf Figur 5 leicht abgewandeltes Ausführungsbeispiel eines Verschlusses auf einer Pulverspritze in aktiviertem Zustand;
- Figur 7: eine Schnittansicht des Ausführungsbeispiels gemäß Figur 6 entlang der Linie A-A;
- Figur 8: ein viertes Ausführungsbeispiel eines Verschlusses;
- Figur 9: das Ausführungsbeispiel gemäß Figur 8 in aktiviertem Zustand;
- Figur 10: ein fünftes Ausführungsbeispiel eines Verschlusses, und
- Figur 11: das Ausführungsbeispiel gemäß 10 in aktiviertem Zustand.

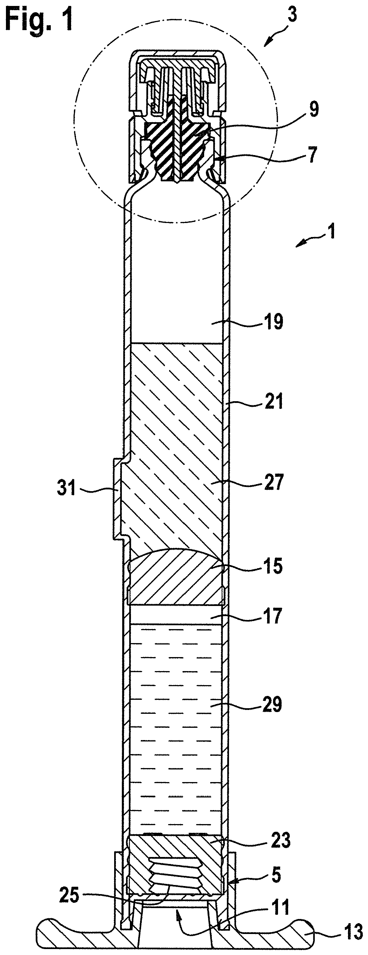

Figur 1 zeigt eine Pulverspritze 1, die mit einem ersten Ausführungsbeispiel eines Verschlusses 3 versehen ist. Die Pulverspritze 1 weist ein proximales Ende 5 und ein distales Ende 7 auf. An dem distalen Ende 7 ist eine distale Öffnung 9 vorgesehen. An dem proximalen Ende 5 ist eine proximale Öffnung 11 angeordnet. Im Bereich der proximalen Öffnung 5 ist eine Fingerauflage 13 angeordnet, welche einen Durchlass für eine nicht dargestellte Kolbenstange aufweist.

Bei dem dargstellten Ausführungsbeispiel ist die Pulverspritze 1 als Doppelkammersystem ausgebildet. Bei dessen Herstellung wird bevorzugt durch die proximale Öffnung 11 ein Mittelstopfen 15 in das Innere der Pulverspritze 1 eingeführt, welcher eine proximale Kammer 17 von einer distalen Kammer 19 trennt. Die distale Kammer 19 wird bevorzugt durch die distale Öffnung 9 befüllt. Beispielsweise kann hier ein gelöster Wirkstoff oder eine Kombination gelöster Wirkstoffe, allgemein also eine Lösung eingefüllt werden, welche anschließend lyophilisiert wird. Danach wird die distale Kammer 19 beziehungsweise die distale Öffnung 9 durch den Verschluss 3 verschlossen. Ein in der distalen Kammer 19 angeordneter LyophilisatKuchen haftet typischerweise an einer Wandung 21 der Pulverspritze 1, so dass er nicht frei in der distalen Kammer 19 beweglich ist.

Wird stattdessen eine pulverförmige, lösliche Substanz in die distale Kammer 19 gefüllt, kann diese sich dort frei verteilen. Die distale Kammer 19 wird mit dem Verschluss 3 verschlossen. Das Pulver kann dann in den Bereich eines Kanals des Verschlusses 3 gelangen und dort gegebenenfalls unlösliche Agglomerate bilden. Diese verstopfen den Kanal und beeinträchtigen die Funktionsfähigkeit der Pulverspritze 1.

Durch die proximale Öffnung 11 kann in die proximale Kammer 17 ein Lösungsmittel eingefüllt werden, wonach diese durch einen Endstopfen 23 verschlossen wird. Der Endstopfen 23 weist bevorzugt Kopplungsmittel 25 zur Kopplung mit einer nichtdargestellten Kolbenstange auf. Bei dem dargestellten Ausführungsbeispiel ist das Kopplungsmittel 25 ein Innengewinde, welches mit einem an der nicht dargestellten Kolbenstange vorgesehenen Außengewinde kämmen kann.

Bevorzugt liegt also in der distalen Kammer 19 der hier als Doppelkammersystem ausgebildeten Pulverspritze 1 ein Pulver 27 vor. In der proximalen Kammer 17 liegt bevorzugt ein Lösungsmittel 29 vor. Das Pulver 27 ist bevorzugt in dem Lösungsmittel 29 löslich.

Die Wandung 21 weist im Bereich der distalen Kammer 19 einen radialen Vorsprung auf, der sich - in Umfangsrichtung gesehen - nur über einen relativ kleinen Windelbereich erstreckt und als Bypass 31 ausgebildet ist. Zur Aktivierung des Doppelkammersystems wird der Endstopfen 23 mit Hilfe der hier nicht dargestellten Kolbenstange in Richtung des distalen Endes 7 verlagert, wobei sich aufgrund der in der proximalen Kammer 17 ausgebildeten Druckkräfte auch der M ittelstopfen 15 in diese Richtung verlagert.

In der vorliegenden Beschreibung wird allgemein Bezug genommen auf eine Längsrichtung, welche der Längserstreckung der Pulverspritze 1 entspricht. Eine radiale Richtung ist entsprechend eine Richtung, die auf der Längsrichtung senkrecht steht. Die Längsrichtung wird auch als axiale Richtung angesprochen. Eine Umfangsrichtung erstreckt sich entlang einer Umfangslinie um die Längsachse der Pulverspritze 1.

Der Bypass 31 weist - in Längsrichtung gesehen - eine Erstreckung auf, die größer ist als die axiale Länge des Mittelstopfens 15. Wird daher der Mittelstopfen 15 in den Bereich des Bypasses 31 verlagert, wird eine Fluidverbindung zwischen der proximalen Kammer 1 7 und der distalen Kammer 19 über den Bypass 31 ausgebildet. Das Lösungsmittel 29 wird dann insbesondere mittels einer weiteren Verlagerung des Endstopfens 23 von der proximalen Kammer 17 in die distale Kammer 19 eingebracht, wo es das Pulver 27 löst. Schließlich wird ein Zustand erreicht, in dem der Mittelstopfen 15 und der Endstopfen 23 aneinander anliegen. Durch eine Weiterverlagerung der beiden Stopfen zu dem distalen Ende 7 hin kann die nunmehr in der distalen Kammer 19 vorliegende Lösung aus der Pulverspritze 1 ausgetrieben und vorzugsweise in einen Patienten injiziert werden. Die Ausgestaltung und Funktionsweise solcher Doppelkammersysteme ist an sich bekannt, sodass hier nicht weiter darauf eingegangen wird.

Bei Doppelkammersystemen, die in ihrer distalen Kammer Pulver aufnehmen sollen, ist die distale Öffnung 9 bevorzugt im Vergleich zu für Lyophilisate vorgesehenen Doppelkammersystemen erweitert, weil so das Pulver leichter eingefüllt werden kann, während für eine Lösung ein kleinerer Durchmesser genügt.

Die Erfindung ist nicht auf Pulverspritzen beschränkt, die als Doppelkammersysteme ausgebildet sind. Das Problem, dass in einer Kammer der Spritze vorhandenes Pulver in einem Kanal eines Verschlusses agglomerieren kann, ergibt sich grundsätzlich bei jeder Pulverspritze. Entsprechend ist auch die hier vorgeschlagene Lösung bei jeder beliebigen Pulverspritze anwendbar. Es zeigt sich auch, dass es nicht ausgeschlossen ist, dass ein in einer distalen Kammer eines Doppelkammersystems vorliegendes Lyophylisat im Laufe der Lagerung zumindest teilweise pulverförmig wird und dann den Kanal eines Verschlusses verstopfen kann. Der hier vorgeschlagene Verschluss kann daher selbstverständlich bevorzugt auch bei einem Doppelkammersystem verwendet werden, welches in seiner distalen Kammer ein Lyophilisat umfasst.

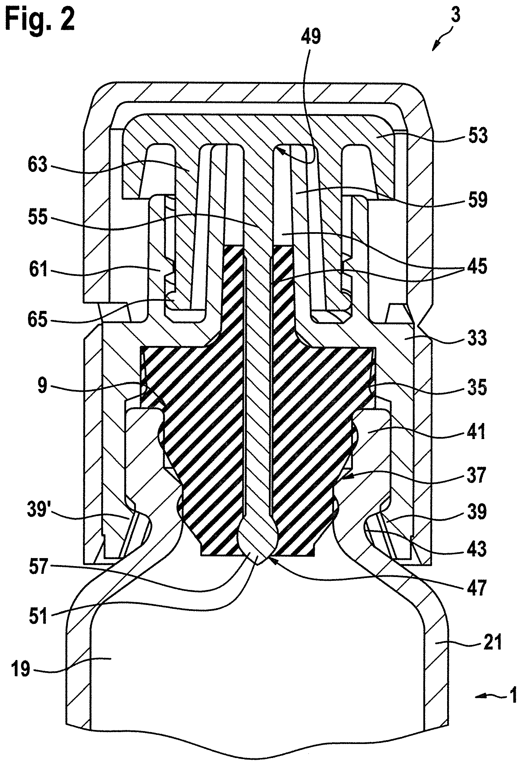

Figur 2 zeigt eine Detailansicht des Ausführungsbeispiels des Verschlusses 3 gemäß Figur 1. Gleiche und funktionsgleiche Elemente mit sind mit gleichen Bezugszeichen versehen, sodass insofern auf die vorangegangene Beschreibung verwiesen wird. Der Verschluss 3 weist einen Grundkörper 33 auf. An diesem ist ein Dichtelement 35 so angeordnet, dass es an der distalen Öffnung 9 der Pulverspritze 1 in der hier dargestellten Verschließposition des Verschlusses 3 auf der Pulverspritze 1 dichtend anliegt.

Das Dichtelement 35 besteht aus einem elastischen Material und ist quasi zwischen der Wandung 21 der Pulverspritze 1 und dem Grundkörper 33 des Verschlusses 3 eingeklemmt, sodass sich eine Dichtwirkung ergibt. Dabei weist bei dem dargestellten Ausführungsbeispiel ein Mündungsbereich 37 der Pulverspritze 1 eine Innenkontur auf, an die eine Außenkontur des Dichtelements 35 angepasst ist, sodass dieses in der Verschließposition des Verschlusses 3 entlang des gesamten Mündungsbereichs 37 dichtend anliegt.

In Figur 2 sind bereichsweise Kanten des Dichtelements 35 mit Kanten des Grundkörpers 33 und der Wandung 21 überlappend dargestellt. Dies ist lediglich ein Artefakt des Zusammenbaus in der technischen Zeichnung, in welcher das Dichtelement 35 in seinem nichtkomprimierten Zustand zwischen dem Grundkörper 33 und der Wandung 21 dargestellt ist. In Wirklichkeit wird das Dichtelement zusammengepresst und liegt dann dicht an dem Grundkörper 33 und der Wandung 21 an. Wäre dieser Zustand hier realistisch dargestellt, würden die entsprechenden Kanten aneinander anliegen, sodass es keine Überlappungen gäbe.

Der Grundkörper 33 weist an seinem der distalen Kammer 19 zugewandten Ende nach innen weisende, radiale Vorsprünge 39, 39' auf. Mit diesen hintergreift er einen Flansch 41 der Pulverspritze 1, der im Mündungsbereich 37 so vorgesehen ist, dass in der Wandung 21 quasi eine Hinterschneidung oder Nut 43 ausgebildet ist. In diese greifen die Vorsprünge 39, 39' in der Verschließposition des Verschlusses 3 quasi als Rastnasen ein. So ist es möglich, in das Dichtelement 35 Kräfte einzuleiten, die es zusammenpressen und seine dichte Anlage im Bereich der distalen Öffnung 9 beziehungsweise im Mündungsbereich 37 ermöglichen. Zugleich wird der Verschluss 3 durch die Vorsprünge 39, 39' sicher auf dem durch die Wandung 21 gebildeten Grundkörper der Pulverspritze 1 gehalten.

Wird der Verschluss 3 in Zusammenhang mit einem in der distalen Kammer 19 vorgesehenen Lyophilisat verwendet, umfasst bevorzugt der Flansch 41 eine ringförmige Nut, die ihn - in Umfangsrichtung gesehen - umgreift. Auch in diese hier nicht dargestellte Nut können die Vorsprünge 39, 39' verrastend eingreifen. In der Ansicht von Figur 2 ist dann quasi über der dargestellten Rastposition des Verschlusses 3 eine weitere Rastposition ausgebildet, in welcher das Dichtelement 35 nicht nur nicht komprimiert ist, sondern einen Spalt im Bereich der distalen Öffnung 9 freilässt, sodass eine Fluidverbindung von der distalen Kammer 19 in den Umgebungsbereich der Pulverspritze 1 besteht. Es ist möglich, eine in der distalen Kammer 19 vorgelegte Lösung zu lyophilisieren, während der Verschluss 3 in seiner oberen Rastposition angeordnet ist. Nach Abschluss des Lyophilisationsvorgangs wird der Verschluss 3 dann in seine in Figur 2 dargestellte Rastposition gebracht, in welcher er die distale Kammer 19 dichtend verschließt.

Der Verschluss 3 weist einen Kanal 45 auf, welcher den Grundkörper 33 und das Dichtelement 35 durchsetzt. Der Kanal 45 weist ein proximales Ende 47 und ein distales Ende 49 auf.

Bei bekannten Verschlüssen ist es möglich, dass pulverförmiges Material aus der distalen Kammer 19 über das proximale Ende 47 in den Kanal 45 eindringen kann. Dort können sich Agglomerate bilden, die gegebenenfalls unlöslich sind und die Funktionsfähigkeit der Pulverspritze 1 beeinträchtigen.

Um dies zu vermeiden, weist der Verschluss 3 ein Rückhalteelement 51 auf, das so ausgebildet und/oder anordenbar ist, dass jedenfalls vor einer Aktivierung der Pulverspritze vorzugsweise kein Pulver aus der distalen Kammer 19 in den Kanal 45 eindringen kann, wenn der Verschluss 3 in seiner in Figur 2 dargestellten Verschließposition auf der Pulverspritze angeordnet ist.

Bei dem dargestellten Ausführungsbeispiel weist der Verschluss 3 eine Verschlusskappe 53 auf, die das distale Ende 49 des Kanals 45 verschließt.

Die Verschlusskappe 53 umfasst einen vorzugsweise stabförmigen Vorsprung 55. Dieser durchsetzt den Kanal. Das Rückhalteelement 51 ist an dem Vorsprung 55 vorgesehen.

Beispielsweise ist es möglich, den vorzugsweise stabförmigen Vorsprung 55 mit einem Durchmesser auszubilden, der größer ist als der Durchmesser des das Dichtelement 35 durchsetzenden Abschnitts des Kanals 45. Das Dichtelement 35 wird dann komprimiert, wenn der Vorsprung 55 in den entsprechenden Kanalabschnitt eingeführt wird, und es liegt dementsprechend dichtend an demselben an. Weist der Vorsprung 55 dann - in Längsrichtung gesehen - eine Erstreckung auf, die bis zum proximalen Ende 47 reicht, ist der Kanal 45 dicht verschlossen, sodass kein Pulver in ihn eindringen kann.

Nachteilig an einem Ausführungsbeispiel, bei dem der stabförmige Vorsprung 55 entlang seiner gesamten Längserstreckung einen entsprechenden Durchmesser aufweist ist allerdings, dass zur Entfernung der Verschlusskappe 53 große Kräfte aufgrund der Reibung zwischen dem Vorsprung 55 und dem Dichtelement 35 nötig sind. Es wird daher ein Ausführungsbeispiel bevorzugt, bei dem das Rückhalteelement 51 als Verdickung 57 des stabförmigen Vorsprungs ausgebildet ist. Bei dem dargestellten, bevorzugten Ausführungsbeispiel ist die Verdickung 57 an einem der distalen Kammer 19 zugewandten Ende des Vorsprungs 55 vorgesehen. Bevorzugt durchgreift der Vorsprung 55 das gesamte Dichtelement 35, sodass er mit der Verdickung 57 zumindest bereichsweise aus dem proximalen Ende 47 des Kanals 45 herausragt. Im Bereich der Verdickung 57, die vorzugsweise einen größten Durchmesser aufweist, der größer ist als der Innendurchmesser des in dem Dichtelement 35 vorgesehenen Abschnitts des Kanals 45, liegt das Dichtelement 35 dichtend an, sodass das proximale Ende 47 dicht verschlossen ist. Es kann also kein Pulver in den Kanal 45 eindringen.

Ist ein Rückhalteelement in Form der Verdickung 57 an dem Vorsprung 55 vorgesehen, kann dessen Durchmesser außerhalb der Verdickung 57 kleiner ausgebildet sein, als es dem Innendurchmesser des Abschnitts des Kanals 45 in dem Dichtelement 35 entspricht. Hierdurch werden Reibungskräfte beim Entfernen der Verschlusskappe 53 reduziert.

Es ist möglich, den Durchmesser des Vorsprungs 55 in dem Bereich, in dem dieser dem proximalen Ende 47 gegenüberliegend aus dem Dichtelement 35 austritt, größer zu gestalten, um auch hier den Abschnitt des Kanals 45, der sich durch das Dichtelement 35 erstreckt, dicht zu verschließen. In diesem Fall ist dann der Eintritt und der Austritt des Kanals 45 in das Dichtelement 35 beziehungsweise aus diesem heraus durch den Vorsprung 55 dicht verschlossen. Anhand von Figur 2 zeigt sich noch Folgendes: Der Grundkörper 33 weist bevorzugt einen Ansatz 59 auf, welcher der Kopplung mit einer Kanüle oder Spritzennadel dient. Insbesondere ist es möglich, den Ansatz 59 konisch auszubilden. Besonders bevorzugt ist er als Luer-Konus ausgebildet. Entsprechend ist es möglich, dass der Grundkörper 33 auch ein Luer-Gewinde 61 aufweist, welches den Ansatz 59 umgreift und der Kopplung mit Abgabemitteln zur Abgabe eines Wirkstoffs oder einer Lösung dient. Ein Luer-Konus und ein Luer-Gewinde sind bekannt, sodass hier nicht näher darauf eingegangen wird. Bevorzugt weist die Verschlusskappe 53 noch einen den Ansatz 59 umgreifenden Wandabschnitt 63 auf. An dessen der distalen Kammer 19 zugewandten Ende ist ein sich - in Umfangsrichtung gesehen - erstreckender radialer Vorsprung 65 vorgesehen. Dieser steht bevorzugt in Eingriff mit dem Luer-Gewinde 61, sodass die Verschlusskappe 53 in das Luer-Gewinde ein- beziehungsweise aus diesem herausgeschraubt werden kann.

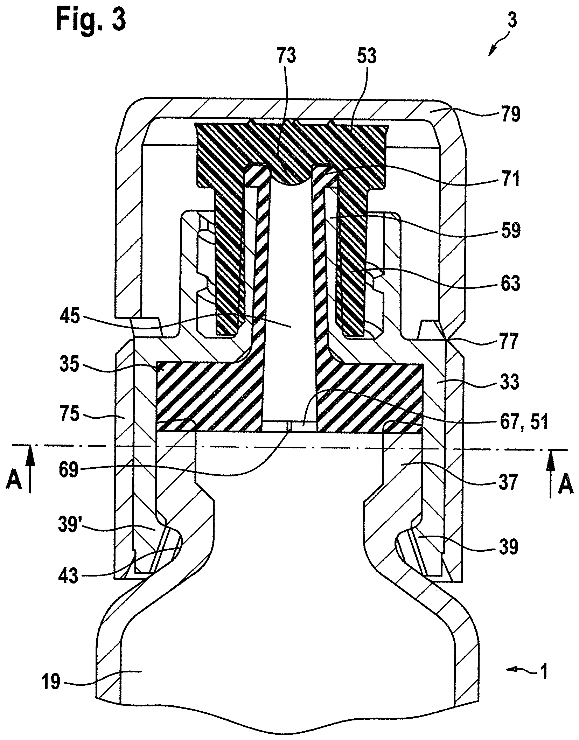

Figur 3 zeigt ein zweites Ausführungsbeispiel eines Verschlusses 3. Gleiche und funktionsgleiche Elemente sind mit gleichen Bezugszeichen versehen, sodass insofern auf die vorangegangene Beschreibung verwiesen wird. Zur besseren Übersicht werden in den Figuren nicht stets alle Bezugszeichen aufgeführt, sondern nur solche, die in der Beschreibung auch unmittelbar erwähnt werden. Bei dem in Figur 3 dargestellten Ausführungsbeispiel ist das Rückhalteelement als Membran 67 ausgebildet. Diese kann prinzipiell durchstechbar, zerreißbar, insbesondere durch Druckkräfte aufsprengbar oder in anderer Weise zerstörbar ausgebildet sein. Dabei haben zerstörbare, insbesondere durchstechbare oder aufsprengbare Membranen den Nachteil, dass sich kleine Partikel beim Öffnen von der Membran lösen und in den Patienten injiziiert werden können. Bei dem dargestellten Ausführungsbeispiel ist die Membran 67 daher bevorzugt als geschlitzte Membran ausgebildet. Sie weist also mindestens einen Schlitz 69 auf, in dessen Bereich sie aufgrund von Druckkräften aufgeweitet werden kann. Der Schlitz kann in einer vorzugsweise spritzgegossenen Membran urgeformt, also beim Spritzen erzeugt werden. Er ist bevorzugt auch möglich, den Schlitz nach der Herstellung in diese einzubringen, beispielsweise durch Schneiden, bevorzugt Laserschneiden. Bevorzugt ist der Schlitz in geschlossenem Zustand so eng, dass kein Pulver hindurchdringen kann. Es ist aber auch möglich, dass nur so wenig Pulver durch den Schlitz dringen kann, dass eine Agglomeratbildung in dem Kanal 45 ausgeschlossen ist. Auch ein solches Ausführungsbeispiel ist vom Gegenstand der Erfindung umfasst.

Bei dem dargestellten Ausführungsbeispiel ist das Dichtelement 35 - in axialer Richtung gesehen - kürzer ausgebildet als bei dem Ausführungsbeispiel gemäß Figur 2. Insbesondere weist es hier keine Kontur auf, die sich in den Mündungsbereich 37 hineinerstreckt und dessen Kontur folgt. Gleichwohl ist es auch möglich, bei einem Dichtelement, welches wie in Figur 2 ausgebildet ist, eine vorzugsweise geschlitzte Membran vorzusehen.

Das Dichtelement 35 erstreckt sich bei dem dargestellten Ausführungsbeispiel allerdings durch den gesamten Ansatz 59 des Grundkörpers 33. Es übergreift sogar - in axialer Richtung gesehen - den Ansatz 59 und bildet an dessen distalem Ende einen ringförmigen Auflagebereich 71.

Die Verschlusskappe 53 liegt hier mit dem Wandabschnitt 63 dichtend an dem Ansatz 59 an. Sie weist nur einen kurzen zentralen Vorsprung 73 auf, der vergleichweise nur über eine kurze Distanz in den Kanal 45 hineinragt und dicht an dem Dichtelement 35 anliegt.

Das Material, welches der Grundkörper 33 und damit auch der Ansatz 59 umfasst, beziehungsweise aus welchem diese bestehen, ist typischerweise nicht für einen Primärkontakt geeignet. Dies bedeutet, dass ein Medikament insbesondere während seiner Lagerung keinen Kontakt zu dem Grundkörper 33 haben darf. Daher weist typischerweise die Verschlusskappe 53 einen Vorsprung 73 auf, der sich - in axialer Richtung gesehen - zumindest so weit erstreckt, dass er mit dem Dichtelement 35 dichtend abschließt. Hierdurch wird vermieden, dass ein in der distalen Kammer 19 vorgesehener Wirkstoff Kontakt zu dem Material des Grundkörpers 33 bekommt. Erstreckt sich das Dichtelement 35 aber - wie bei dem in Figur 3 dargestellten Ausführungsbeispiel - durch den gesamten Ansatz 59 hindurch und bildet sogar jenseits von diesem einen ringförmigen Auflagebereich 71, ist es nicht nötig, einen langen Vorsprung 73 an der Verschlusskappe 53 vorzusehen. Stattdessen genügt es, wenn dieser so ausgebildet ist, dass er noch dichtend mit dem Auflagebereich 71 in Eingriff kommt. Es ist dann gewährleistet, dass eine in der distalen Kammer 19 vorgesehene Substanz nicht mit dem Material des Grundkörpers 33 in Kontakt kommt.

Es zeigt sich auch noch Folgendes: Die Vorsprünge 39, 39' werden in der Verschließposition des Verschlusses 3 durch eine Sicherungskappe 75 in ihre Rastposition in der Nut 43 gedrängt. Der Sicherungsring 75 ist über Abreißstege 77 mit einer Sicherungskappe 79 verbunden, welche die Verschlusskappe 53 über- und umgreift. Beim Aufsetzen des Verschlusses 53 wird zunächst der Grundkörper 33 in seine Verschließposition verlagert, anschließend wird der Sicherungsring 75 mit der Sicherungskappe 79 über denselben geschoben, sodass der Grundkörper 33 letztlich sicher in seiner verrasteten Position gehalten wird. Um den Verschluss 3 zu öffnen, wird die Sicherungskappe 79 im Bereich der Abreißstege 77 von dem Sicherungsring 75 getrennt und entfernt. Anschließend kann die Verschlusskappe 53 entfernt werden, um eine Fluidverbindung der Umgebung der Pulverspritze 1 mit dem Kanal 45 herzustellen.

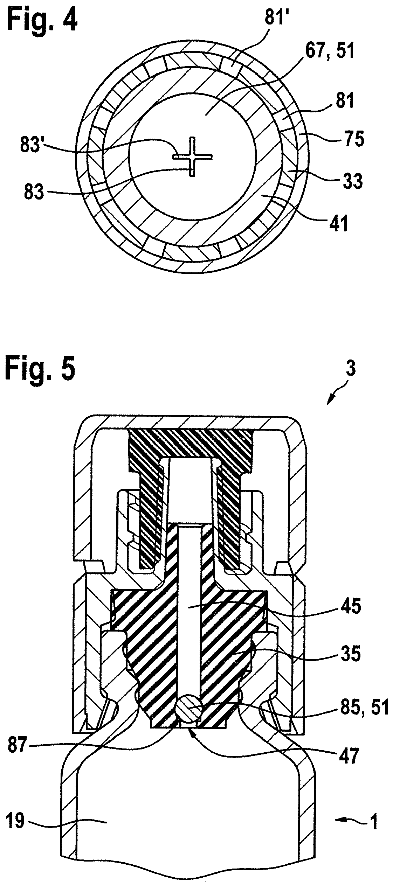

Figur 4 zeigt eine Schnittansicht des Ausführungsbeispiels gemäß Figur 3 entlang der Linie A-A. Gleiche und funktionsgleiche Elemente sind mit gleichen Bezugszeichen versehen, sodass insofern auf die vorangegangene Beschreibung verwiesen wird. Im äußeren Bereich ist der Sicherungsring 75 dargestellt, der den Grundkörper 33 umgreift. Hierbei wird deutlich, dass der Grundkörper 33 im Bereich der Schnittebene und insbesondere auch im Bereich der Vorsprünge 39, 39' - in Umfangsrichtung gesehen - mehrere Schlitze beziehungsweise Ausnehmungen aufweist, welche sich - in axialer Richtung gesehen - über einen gewissen Bereich erstrecken. Rein beispielhaft sind hier zwei Schlitze mit den Bezugszeichen 81, 81' bezeichnet. Insgesamt sind in Figur 4 entlang des Umfangs des Grundkörpers acht Schlitze beziehungsweise Ausnehmungen vorgesehen. Diese Ausnehmungen bewirken zum einen, dass der Grundkörper 33 im Bereich der Vorsprünge 39, 39' eine hinreichende Elastizität aufweist, um über den Flansch 41 geschoben zu werden, bevor die Vorsprünge 39, 39' in die Nut 43 eingreifen. Zum anderen sind die Ausnehmungen dann wesentlich, wenn der Verschluss 3 zusammen mit einem Doppelkammersystem verwendet wird, in dessen distaler Kammer 19 ein Lyophilisat vorgesehen wird. Wie bereits ausgeführt, ist der Verschluss 3 dann während der Lyophilisation vorzugsweise in seiner oberen Rastposition angeordnet, in welcher er die distale Öffnung 9 des Doppelkammersystems nicht dicht verschließt. Der Fluidpfad, über den das bei dem Gefriertrocknungsvorgang verdampfende Lösungsmittel aus der distalen Kammer 19 und die distale Öffnung 9 in die Umgebung des Doppelkammersystems gelangen kann, führt dann durch die Ausnehmungen, insbesondere auch durch die hier beispielhaft bezeichneten Schlitze 81, 81'.

Im Zentrum der Darstellungen von Figur 4 ist die Membran 67 angeordnet. Sie weist zwei Schlitze 83, 83' auf, die aufeinander senkrecht stehen, sodass sich insgesamt eine kreuzförmige Einschlitzung ergibt. Es ist möglich, dass die Membran 67 nur einen Schlitz aufweist. Auch sind mehr als zwei Schlitze möglich. Wenn zwei Schlitze vorgesehen sind, müssen diese keinesfalls senkrecht aufeinanderstehen. Grundsätzlich ist eine beliebige Anzahl und geometrische Anordnung von Schlitzen möglich.

Wesentlich ist, dass eine Breite der Schlitze beziehungsweise eine im Zentrum der hier kreuzförmig angeordneten Schlitze 83, 83' angeordnete Öffnung kleiner ist als eine mittlere Korngröße des Pulvers, welches in der distalen Kammer 19 vorgesehen ist. Bevorzugt ist die Öffnung beziehungsweise die Schlitzbreite kleiner als die kleinste Korngröße dieses Pulvers. Auf diese Weise ist gewährleistet, dass die Membran 67 als Rückhaltemittel 51 wirkt und effizient verhindert, dass Pulver in den Kanal 45 eindringen kann. Wird die Pulverspritze aktiviert, soll also eine dann in der distalen Kammer 19 vorliegende Lösung injiziert werden, wird die Membran 67 aufgrund der dann in der distalen Kammer 19 aufgebauten Druckkräfte aufgeweitet und gibt zumindest im Bereich der Schlitze 83, 83' einen Fluidpfad zum Kanal 45 frei. Es ist auch möglich, dass die Membran 67 unter Einwirkung der Druckkräfte, vorzugsweise entlang der Schlitze 83, 83' aufreißt, sodass ein Fluidpfad mit größerem Durchmesser freigegeben wird.

Bei dem dargestellten Ausführungsbeispiel ist die Membran 67 einstückig mit dem Dichtelement 35 ausgebildet. Es ist auch möglich, sie als separates Element vorzusehen. In diesem Fall ist sie bevorzugt in geeigneter Weise mit dem Dichtelement 35 verbunden. Besonders bevorzugt ist die Membran 67 an dem proximalen Ende 47 des Kanals 49 angeordnet.

Figur 5 zeigt ein drittes Ausführungsbeispiel eines Verschlusses 3. Gleiche und funktionsgleiche Elemente sind mit gleichen Bezugszeichen versehen, sodass insofern auf die vorangegangene Beschreibung verwiesen wird. Das Rückhalteelement 51 ist hier als entlang des Kanals 45 verlagerbares Element ausgebildet. Bei dem dargestellten Ausführungsbeispiel ist das verlagerbare Element eine Kugel 85. Diese weist einen Durchmesser auf, der größer ist als der Durchmesser des Abschnitts des Kanals 45, der in dem Dichtelement 35 angeordnet ist. Hierdurch ist das proximale Ende 47 durch die Kugel 85 verschließbar. Die Kugel 85 wird durch Reibung in ihrer Verschließposition gehalten.

Bei anderen Ausführungsbeispielen kann das verlagerbare Element eine abweichende Geometrie aufweisen. Beispielsweise ist mindestens ein Vorsprung an der Kugel 85 möglich, der sich über das proximale Ende 47 hinaus in die distale Kammer 19 erstreckt. Auf diese Weise ist der Kanal 45 auch unmittelbar an seinem proximalen Ende 47 verschließbar. Statt der Kugel 85 kann beispielsweise auch ein zylindrisches Element vorgesehen sein. Auch dieses kann einen entsprechenden Vorsprung aufweisen. Beliebige andere Geometrien sind möglich.

Das verlagerbare Element umfasst bevorzugt Glas oder besteht aus diesem. Es weist dann die nötige Härte auf, so dass das Dichtelemente 35 dichtend an ihm anliegen kann. Außerdem ist Glas ein für den Primärkontakt geeignetes Material.

Bei dem dargestellten Ausführungsbeispiel ist am proximalen Ende 47 ein Haltemittel 87 für das verlagerbare Element vorgesehen. Dieses verhindert, dass das verlagerbare Element in die distale Kammer 19 eindringen kann. Bei dem dargestellten Ausführungsbeispiel ist das Haltemittel 87 als radialer Vorsprung einer Wandung des Kanals 45 ausgebildet, an dem die Kugel 85 anliegt. Weist das verlagerbare Element einen Vorsprung auf, kann dieser sich durch den Bereich des Haltemittels 87 hindurch erstrecken, sodass auch dieser Bereich sicher gegen ein Eindringen von Pulver aus der distalen Kammer 1 9 geschützt ist. Ein Restvolumen des Kanals 45, in welchem gegebenenfalls noch Agglomeratbildung möglich ist, wird so nicht nur minimiert, sondern weitestgehend oder sogar vollständig vermieden.

Werden zur Aktivierung der Pulverspritze 1 Druckkräfte in die distale Kammer 19 eingeleitet, verlagert sich das verlagerbare Element in dem Kanal 45 von der distalen Kammer 19 weg. Schließlich tritt es an dem distalen Ende 49 aus dem Kanal 45 aus.

Hierbei ist nachteilig, dass das verlagerbare Element dann entweder aus der Pulverspritze 1 ausgespült wird, wobei es schlimmstenfalls in einen Patienten injiziert werden kann, oder dass es eine Kanüle oder eine Spritzennadel verstopft beziehungsweise deren Einlass verschließt. Es ist daher bevorzugt ein Rückhaltemittel vorgesehen, welche das verlagerbare Element zurückhält und zugleich einen Fluidpfad in eine Umgebung der Pulverspritze 1 freigibt. Ein solches Rückhaltemittel kann in einem speziell angefertigten Kanülenaufsatz vorgesehen sein. Dies ist allerdings eine vergleichsweise aufwendige und kostenintensive Lösung, weil dann der Verschluss 3 oder die Pulverspritze 1 nicht mit herkömmlichen Kanülen beziehungsweise Spritzennadeln verwendet werden kann.

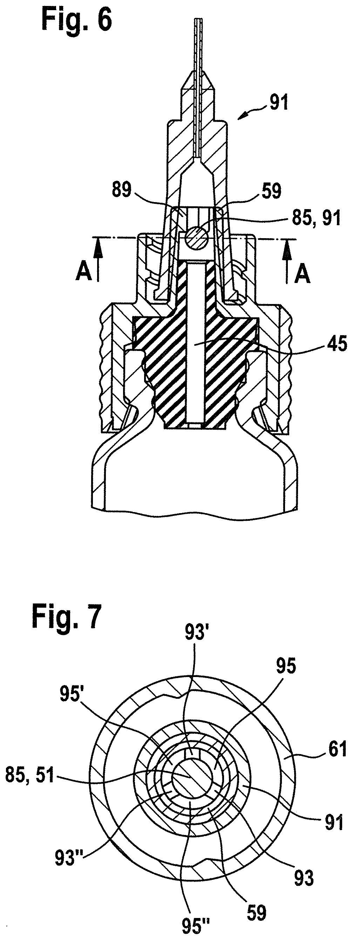

Figur 6 zeigt ein in Hinblick auf Figur 5 leicht abgewandeltes Ausführungsbeispiel des Verschlusses 3 in aktiviertem Zustand. Gleiche und funktionsgleiche Elemente sind mit gleichen Bezugszeichen versehen, sodass insofern auf die vorangegangene Beschreibung verwiesen wird. Hier ist im Bereich des distalen Endes 49 des Kanals 45 ein Rückhaltemittel 89 für das verlagerbare Element vorgesehen. Auf dem Ansatz 59 ist eine Kanüle 91 angeordnet. Diese umfasst vorzugsweise einen Innenkonus, der mit dem vorzugsweise konisch ausgebildeten Ansatz 59 so zusammenwirken kann, dass eine dichte Verbindung von der Pulverspritze 1 zur Kanüle 91 gewährleistet ist. Das Rückhaltemittel 91 fängt quasi das verlagerbare Element auf. Zugleich bleibt aber ein Fluidpfad von dem Kanal 45 zu der Kanüle 91 bestehen. Insbesondere kann dabei eine Injektionslösung das hier als Kugel 85 ausgebildete verlagerbare Element umströmen.

Die Funktionsweise des Rückhaltemittels 89 wird anhand der Schnittdarstellung von Figur 7 entlang der Linie A-A in Figur 6 näher erläutert. Gleiche und funktionsgleiche Elemente sind mit gleichen Bezugszeichen versehen, sodass insofern auf die vorangegangene Beschreibung verwiesen wird. Im äußeren Bereich von Figur 7 ist das Luer-Gewinde 61 dargestellt. Nach innen - in radialer Richtung gesehen - folgt als nächstes ein Wandabschnitt der Kanüle 91. Weiter radial innen ist schließlich der Ansatz 59 dargestellt. Auch hier ergibt sich wieder eine scheinbare Überlappung, die daraus resultiert, dass der zeichnerische Zusammenbau mit den Elementen in ihrer zerlegten Konfiguration vorgenommen wurde. Aufweitungen oder Kompression von Material, die sich beim Zusammensetzen der verschiedenen Elemente ergeben, wurden hier nicht berücksichtigt, sodass sich scheinbar überlappende Bereiche ergeben.

Der Ansatz 59 weist im Bereich des distalen Endes 49 radiale Vorsprünge 93, 93', 93"' auf, die so weit radial nach innen in den Kanal 45 hineinragen, dass das verlagerbare Element beziehungsweise die Kugel 85 von ihnen zurückgehalten wird. Bei dem dargestellten Ausführungsbeispiel sind - in Umfangsrichtung gesehen - drei radiale Vorsprünge 93, 93', 93" vorgesehen. Letztlich ist ein radialer Vorsprung ausreichend, wenn er sich radial weit genug nach innen erstreckt, um die Kugel 85 zurückzuhalten. Es sind auch zwei oder mehr als drei Vorsprünge möglich. Wesentlich ist, dass - in Umfangsrichtung gesehen - Freiräume 95, 95', 95" bestehen bleiben, die eine Fluidverbindung zwischen dem Kanal 45 und einer Umgebung der Pulverspritze 1 beziehungsweise der Kanüle 91 ermöglichen. Eine Injektionslösung kann also die Kugel 85 beziehungsweise das verlagerbare Element umströmen und durch die Freiräume 95, 95', 95" aus dem Kanal 45 aus und in den Umgebungsbereich der Pulverspritze 1 beziehungsweise in die Kanüle 91 eintreten.

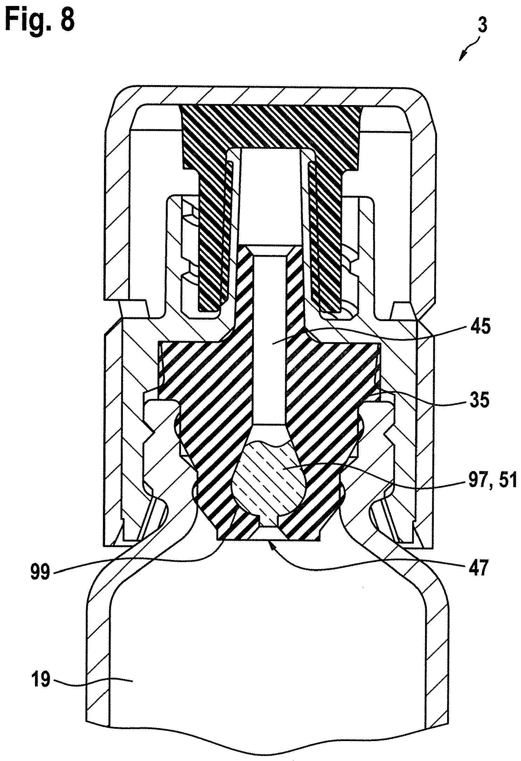

Figur 8 zeigt ein viertes Ausführungsbeispiel des Verschlusses 3. Gleiche und funktionsgleiche Elemente sind mit gleichen Bezugszeichen versehen, sodass insofern auf die vorangegangene Beschreibung verwiesen wird. Das Rückhalteelement umfasst hier eine lösliche Substanz 97. Diese ist so ausgewählt, dass sie in demselben Lösungsmittel löslich ist, in dem auch das aufzulösende Pulver oder gegebenenfalls Lyophilisat löslich ist, welches in der distalen Kammer 19 vorlegt. Besonders bevorzugt ist die lösliche Substanz 97 eine lyophilisierte Substanz. Mithilfe einer hier nicht dargestellten Vorrichtung kann eine Lösung in das Dichtelement 35 eingebracht und dort lyophilisiert werden, um einen den Kanal 45 verschließenden Lyophilisatkuchen zu bilden.

Bei dem dargestellten, bevorzugten Ausführungsbeispiel ist im Bereich des proximalen Endes 47 eine Erweiterung 99 zur Aufnahme der Substanz 97 vorgesehen. Diese bildet hier quasi einen Pfropfen, der den Kanal 45 verschließt und bewirkt, dass in der distalen Kammer 19 vorliegendes Pulver nicht in denselben eindringen kann.

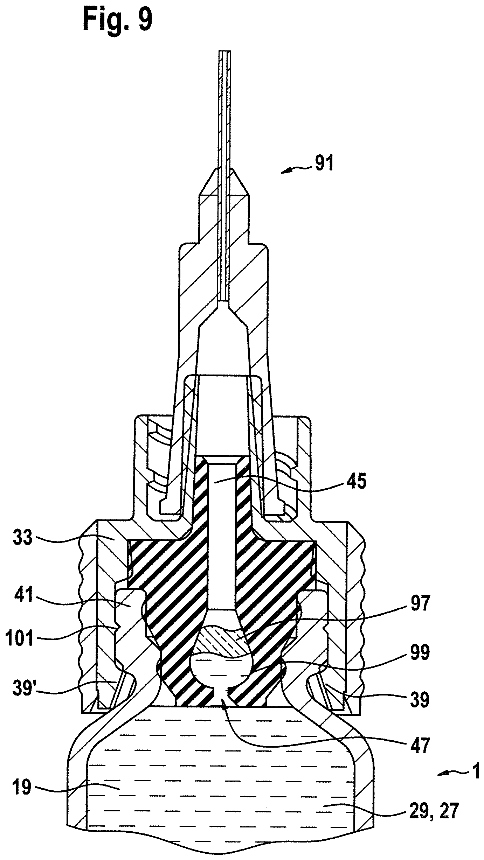

Die Funktionsweise dieses Ausführungsbeispiels wird anhand von Figur 9 näher erläutert. Gleiche und funktionsgleiche Elemente sind mit gleichen Bezugszeichen versehen, sodass insofern auf die vorangegangene Beschreibung verwiesen wird. Wird die Pulverspritze 1 aktiviert, gelangt Lösungsmittel 29 in die distale Kammer 19. Dort löst sich das Pulver 27 in dem Lösungsmittel 29. Die Lösung gelangt dann über das proximale Ende 47 in die Erweiterung 99, wo die Substanz 97 vorliegt. Da diese ebenfalls in dem Lösungsmittel 29 löslich ist, wird sie, wie in Figur 9 dargestellt, aufgelöst. Dies bedeutet, dass sich der den Kanal 45 verschließende Pfropfen nach und nach löst, sodass letztlich die Fluidverbindung von der distalen Kammer 19 zum Kanal 45 freigegeben wird. Schließlich kann die Injektionslösung dann in die Kanüle 91 und weiter in einen Patienten gefördert werden.

Es ist möglich, dass die Substanz 97 Hilfsstoffe wie beispielsweise Vitamin C umfasst. Zusätzlich zu dem gegebenenfalls in der distalen Kammer 19 vorliegenden Wirkstoff können also quasi mithilfe der als Rückhalteelement 51 vorgesehenen Substanz 97 zusätzliche Hilfssubstanzen zugeben werden.

Die Substanz 97 kann auch vorzugsweise als Pellet in das Dichtelement 35, bevorzugt in die Erweiterung 99 eingebracht werden. In diesem Fall kann der zusätzlich Lyophilisierungsschritt für die Substanz 97 entfallen.

In Figur 9 zeigt sich noch Folgendes: Hier ist beispielhaft eine Ringnut 101 im Bereich des Flanschs 41 dargestellt, in welcher die Vorsprünge 39, 39' des Grundkörpers 33 in dessen oberer Rastposition einrasten, wenn eine Substanz in der distalen Kammer 19 lyophilisiert wird.

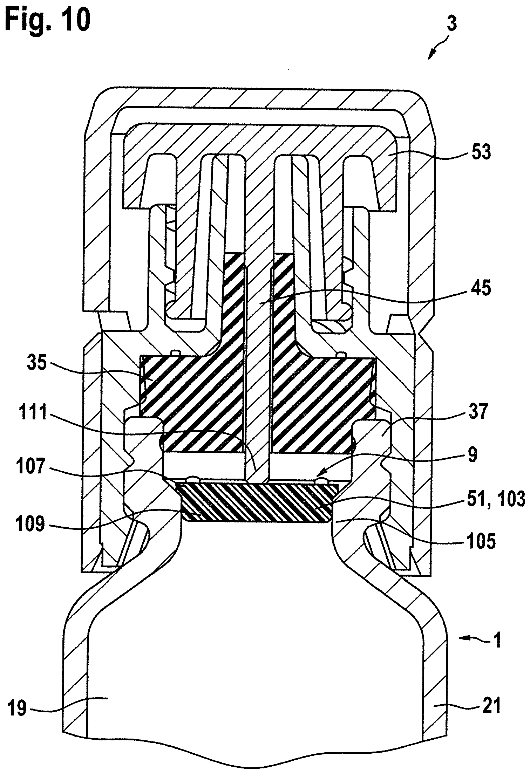

Figur 10 zeigt ein fünftes Ausführungsbeispiel des Verschlusses 3. Gleiche und funktionsgleiche Elemente sind mit gleichen Bezugszeichen versehen, sodass insofern auf die vorangegangene Beschreibung verwiesen wird. Das Rückhalteelement 51 umfasst hier eine Verschlussscheibe 103. Die Verschlussscheibe 103 kann so angeordnet werden, dass sie die distale Kammer 19 verschließt, sodass kein Pulver in den Kanal 45 gelangen kann.

Die Pulverspritze 1 umfasst in dem Mündungsbereich 37 vorzugsweise eine Verengung 105. Die Wandung 21 springt in diesem Bereich radial nach innen, sodass die Verengung 105 gebildet wird. Bevorzugt ist die Verschlussscheibe 103 so ausgebildet, dass sie an der Verengung 105 dichtend anliegen kann. Hierzu weist sie vorzugsweise einen sich - in Umfangsrichtung gesehen - erstreckenden, radialen Vorsprung 107 auf. In einem sich - in axialer Richtung gesehen - an den Vorsprung 107 anschließenden Bereich 109 weist die Verschlussscheibe 103 bevorzugt einen Außendurchmesser auf, welcher dem Innendurchmesser der Verengung 105 entspricht. Damit folgt die Außenkontur der Verschlussscheibe 103 quasi der Innenkontur der Verengung 105 beziehungsweise des Mündungsbereichs 37, sodass sie mit dem Vorsprung 107 und dem Bereich 109 dichtend an der Wandung 21 anliegt.

Auch bei dem dargestellten Ausführungsbeispiel umfasst die Verschlusskappe 53 vorzugsweise einen stabförmigen Vorsprung, der hier als Sicherungsstift 111 ausgebildet ist und den Kanal 45 durchsetzt. Dieser liegt bei aufgesetzter Verschlusskappe 53 in der Verschließposition des Verschlusses 3 an der Verschlussscheibe 103 an und hält diese in dichtender Anlage an der Verengung 105.

Die Verengung 105 ist bevorzugt im Bereich des distalen Endes 9 der Pulverspritze 1 vorgesehen.

Die Verschlussscheibe 103 umfasst bevorzugt Gummi oder TPE, besonders bevorzugt besteht sie aus mindestens einem dieser Materialien. Sie hat dann sehr gute Dichtungseigenschaften, außerdem sind die genannten Materialien für den Primärkontakt geeignet.

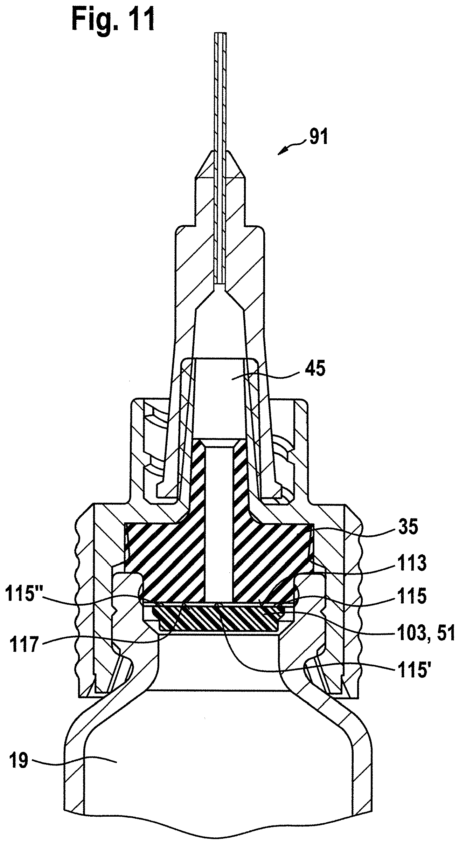

Figur 11 zeigt das Ausführungsbeispiel gemäß Figur 10 in aktiviertem Zustand. Gleiche und funktionsgleiche Elemente sind mit gleichen Bezugszeichen versehen, sodass insofern auf die vorangegangene Beschreibung verwiesen wird. Zur Aktivierung der Pulverspritze 1 wird zunächst die Verschlusskappe 53 und damit auch der Sicherungsstift 111 entfernt. Anschließend wird in der distalen Kammer 19 ein Druck aufgebaut, der die Verschlussscheibe 103 zu dem Dichtelement 35 hindrängt. Diese weist an einer dem Dichtelement 35 zugewandten Fläche 113 mindestens einen Vorsprung, hier drei Vorsprünge 115, 115', 115" auf. Mit diesen stützt sie sich an einer Gegenfläche 117 des Dichtelements 35 ab. Durch die Vorsprünge 115 werden also die Fläche 113 und die Gegenfläche 117 in einem Abstand voneinander gehalten, sodass zwischen ihnen und zwischen den Vorsprüngen 115, 115', 115" ein Fluidpfad ausgebildet wird. Die aus der distalen Kammer 19 austretende Lösung kann daher die Verschlussscheibe 103 umströmen und in den Kanal 45 eintreten. Von dort kann sie wiederum an die Kanüle 91 abgegeben werden.

Insgesamt zeigt sich, dass der hier vorgeschlagene Verschluss und die hier vorgeschlagene Pulverspritze mit Hilfe des Rückhalteelements 51 die Gefahr einer Agglomeratbildung in dem Kanal 45 vermeiden. Dies trägt erheblich dazu bei, die Lagerfähigkeit insbesondere von als Doppelkammersystemen ausgebildeten Pulverspritzen zu verbessern und deren Funktionsfähigkeit auch bei langer Lagerung sicher zu gewährleisten.

## Patentansprüche

1. Verschluss für eine Pulverspitze (1) mit
- einem Grundkörper (33),
- einem Dichtelement (35), welches an dem Grundkörper (33) so angeordnet ist, dass es an einer distalen Öffnung (9) einer Pulverspritze (1) dichtend anliegt, wenn der Verschluss (3) auf der Pulverspritze (1) in seiner Verschließposition angeordnet ist, und
- einem Kanal (45), der den Grundkörper (33) und das Dichtelement (35) durchsetzt und ein proximales und ein distales Ende (47,49) aufweist,
- einer Verschlusskappe (53), die das distale Ende (49) des Kanals (45) verschließt,
**gekennzeichnet durch**
ein Rückhalteelement (51), das so ausgebildet und/oder anordenbar ist, dass vor einer Aktivierung der Pulverspritze (1) ein Eindringen von Pulver (27) aus einer Kammer (19) der Pulverspritze in den Kanal (45) zumindest weitgehend, vorzugsweise vollständig verhindert wird, wenn der Verschluss (3) in seiner Verschließposition auf der Pulverspritze (1) angeordnet ist, und **dadurch**, dass das Rückhalteelement (51) eine Verschlussscheibe (103) umfasst, die so ausgebildet ist, dass sie an einer Verengung (105) einer Pulverspritze (1) dichtend anliegen kann, um ein Eindringen von Pulver (27) in den Kanal (45) zu verhindern, und dass die Verschlusskappe (53) einen stabförmigen Vorsprung aufweist, der den Kanal durchsetzt und in der Verschließposition des Verschlusses (3) die Verschlussscheibe (103) in dichtender Anlage mit der Pulverspritze (1) hält.

2. Verschluss nach Anspruch 1, **dadurch gekennzeichnet, dass** die Verschlussscheibe (103) einen sich - in Umfangsrichtung gesehen - erstreckenden, radialen Vorsprung (107) aufweist.

3. Verschluss nach einem der Ansprüche 1 oder 2, **dadurch gekennzeichnet, dass** die Verschlussscheibe (103) an einer dem Dichtelement (35) zugewandten Fläche (113) mindestens einen Vorsprung (115) aufweist.

4. Pulverspritze mit einer distalen Öffnung (9), **gekennzeichnet durch** einen Verschluss (3) nach einem der Ansprüche 1 bis 3.

5. Pulverspritze nach Anspruch 4, **dadurch gekennzeichnet, dass** die Pulverspritze (1) als Doppelkammersystem ausgebildet ist.

6. Pulverspritze nach einem der Ansprüche 4 oder 5, **dadurch gekennzeichnet, dass** die Pulverspritze (1) eine Verengung (105) im Bereich des distalen Endes (9) aufweist.

## Claims

1. A closure piece for a powder syringe (1) with
- a main body (33),
- a sealing element (35), which is disposed on the main body (33) in such a manner that it rests in a sealing manner against a distal opening (9) of a powder syringe (1), when the closure piece (3) is disposed on the powder syringe (1) in its closing position, and
- a channel (45), which passes through the main body (33) and the sealing element (35) and includes a proximal and a distal end (47,49),
- a closure piece cap (53), which closes the distal end (49) of the channel (45),
**characterized by**
a retaining element (51), which is configured and/or can be disposed such that, prior to an activation of the powder syringe (1), penetration of powder (27), which comes from a chamber (19) of the powder syringe, into the channel (45) is prevented to at least a large extent, preferably completely prevented, when the closure piece (3) is disposed in its closing position on the powder syringe (1), and in that the retaining element (51) comprises a closure piece disc (103) configured such that it is able to rest in a sealing manner against a narrowing (105) of a powder syringe (1) to prevent powder (27) from penetrating into the channel (45) and that the closure piece cap (53) includes a rod-like projection that passes through the channel and holds the closure piece disc (103) in a sealing contact with the powder syringe (1), when the closure piece (3) is in the closing position.

2. Closure piece according to claim 1, **characterized in that** the closure piece disc (103) includes - seen in the circumferential direction - a radially extending protrusion (107).

3. Closure piece according to one of claims 1 or 2, **characterized in that** the closure piece disc (103) has at least one protrusion (115) on a surface (113) that is oriented toward the sealing element (35).

4. A powder syringe having a distal opening (9), **characterized by** a closure piece (3) according to one of the claims 1 to 3.

5. Powder syringe according to claim 4, **characterized in that** the powder syringe (1) is configured as a dual chamber system.

6. Powder syringe according to one of claims 4 or 5, **characterized in that** the powder syringe (1) includes a narrowing (105) in the area of the distal end (9).

## Revendications

1. Fermeture pour une seringue à poudre (1) comprenant
- un corps de base (33),
- un élément d'étanchéité (35) placé de telle façon sur le corps de base (33) qu'il repose en étanchéité sur une ouverture distale (9) d'une seringue à poudre (1), lorsque la fermeture (3) est placée dans sa position de fermeture sur la seringue à poudre (1), et
- un canal (45) qui traverse le corps de base (33) et l'élément d'étanchéité (35) et présente une extrémité proximale et une extrémité distale (47, 49),
- un bouchon de fermeture (53) qui ferme l'extrémité distale (49) du canal (45),
**caractérisée par**
un élément de retenue (51) qui est conçu et/ou peut être agencé de manière à empêcher en au moins grande partie, de préférence complètement, une pénétration de poudre (27) d'une chambre (19) de la seringue à poudre dans le canal (45) avant une activation de la seringue à poudre (1), lorsque la fermeture (3) est agencée dans sa position de fermeture sur la seringue à poudre (1), et en ce que l'élément de retenue (51) comprend une rondelle de fermeture (103) qui est ainsi conçue qu'elle peut se poser en étanchéité sur un resserrement (105) d'une seringue à poudre (1) pour empêcher une pénétration de poudre (27) dans le canal (45), et en ce que le bouchon de fermeture (53) présente une avancée en forme de bâtonnet qui traverse le canal et tient la rondelle de fermeture (103) en position étanche avec la seringue à poudre (1) dans la position de fermeture de la fermeture (3).

2. Fermeture selon la revendication 1, **caractérisée en ce que** la rondelle de fermeture (103) présente, vu dans le sens périphérique, une avancée radial (107) étendu.

3. Fermeture selon l'une des revendications 1 ou 2, **caractérisée en ce que** la rondelle de fermeture (103) présente au moins une avancée (115) sur une surface (113) tournée vers l'élément d'étanchéité (35).

4. Seringue à poudre comprenant une ouverture distale (9), **caractérisée par** une fermeture (3) selon l'une des revendications 1 à 3.

5. Seringue à poudre selon la revendication 4, **caractérisée en ce que** la seringue à poudre (1) est conçue en tant que système à chambre double.

6. Seringue à poudre selon fune des revendications 4 ou 5, **caractérisée en ce que** la seringue à poudre (1) présente un resserrement (105) au niveau de l'extrémité distale (9).
